(19) Europäisches Patentamt
European Patent Office
Office européen des brevets

(11) **EP 3 634 601 B1**

(12) **EUROPEAN PATENT SPECIFICATION**

(45) Date of publication and mention of the grant of the patent:
**11.05.2022 Bulletin 2022/19**

(21) Application number: **18749498.4**

(22) Date of filing: **07.06.2018**

(51) International Patent Classification (IPC):
*B01D 33/06* (2006.01)     *B01D 33/067* (2006.01)
*B01D 33/073* (2006.01)     *B01D 33/46* (2006.01)
*C07C 37/68* (2006.01)

(52) Cooperative Patent Classification (CPC):
(C-Sets available)
**B01D 33/06; B01D 33/067; B01D 33/073;**
**B01D 33/466; C07C 37/82**          (Cont.)

(86) International application number:
**PCT/IB2018/054128**

(87) International publication number:
**WO 2018/225014 (13.12.2018 Gazette 2018/50)**

(54) **ROTARY VACUUM FILTER, METHOD, AND USE**

DREHVAKUUMFILTER, VERFAHREN UND VERWENDUNG

FILTRE ROTATIF À VIDE, PROCÉDÉ ET UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.06.2017 EP 17382350**

(43) Date of publication of application:
**15.04.2020 Bulletin 2020/16**

(73) Proprietor: **SABIC Global Technologies B.V.**
**4612 PX Bergen op Zoom (NL)**

(72) Inventors:
• **BOJARSKI, Aaron David**
  **30390 Cartagena (ES)**
• **IGLESIAS MOYA, Manuel**
  **30390 Cartagena (ES)**
• **CERON MUNOZ, Juan Francisco**
  **30390 Cartagena (ES)**
• **MUNOZ, Gines Cervantes**
  **30390 Cartagena (ES)**

(74) Representative: **Sabic Intellectual Property Group**
**Sabic Intellectual Property Department**
**P.O. Box 3008**
**6160 GA Geleen (NL)**

(56) References cited:
**WO-A1-02/055175      WO-A1-2004/076394**
**DE-A1- 19 961 521    GB-A- 899 165**
**JP-A- 2007 203 184**

(52) Cooperative Patent Classification (CPC): (Cont.)

C-Sets
**C07C 37/82**

**Description**

BACKGROUND

Bisphenol A is commercially produced by the condensation reaction of acetone and two equivalents of phenol in the presence of a catalyst such as an ion-exchange resin. In order to separate the formed bisphenol A from the product mixture, a crystallization step in a crystallization unit is often performed. The resulting bisphenol A-phenol adduct crystals can be separated from the crystallization liquor using a rotary vacuum filter.

DE 199 61 521 to Rainer et al. is directed to a method for the isolation and purification of bis-(4-hydroxyaryl)-alkane/phenol adducts from the acid-catalyzed reaction of ketones with phenols involves separating the crystallized adduct from the mother liquor by continuous filtration in a rotary vacuum filter with several filter cells, washing the crystals and removing the washings by suction.

WO 02-055175 to Daniel is directed to a self-cleaning drum filter. The self-cleaning drum filter comprises a pressure vessel containing a cylindrical filtering drum with a filtering mat and means for removal of the cake collected on the outside of the filtering mat. The means for removal consists of a scanner plate juxtaposed to the inside wall of the pressure vessel. The scanner plate having, along its length, a through-slit centrally placed that provides a passage between the inner and outer faces of said scanner plate, with the outer face being provided, along the length of said slit (22), with a trough (21) which forms, together with the inner wall of said pressure vessel, a channel for collecting the flushing liquid during the cake-removal cleaning operation. GB 899,165 discloses a rotary drum filter of the type having peripheral cells for the collection of the filtrate and a ventilation device for facilitating removal of the filter residue, the ventilation device comprising one or more barrel rings forming part of the filter drum casing, a plurality of ventilation holes in each ring and in communication with said cells, an endless band around each barrel ring to cover the said ventilation holes, and means for causing the band to expose to the atmosphere one or more of said holes in the region of filter residue removing means. JP 2007 203184 discloses a slurry handling method for separating air from a slurry using a rotary vacuum filtering machine constituted so that a rotary drum having a filter cloth provided on its outer peripheral surface under tension is axially mounted on a center pipe and the lower part thereof is immersed in a raw liquid vat to filter the slurry, while the obtained filtrate is led out to the machine by the filtrate pipe connected to the center pipe. The cake formed on the outer surface of the filter cloth is peeled by the air blown from the slit of the valve shoe fixed to the center pipe through a valve bar to be recovered, a gas supply pipe is connected to the slurry supply pipe of the raw liquid vat and air is supplied to the raw liquid vat along with the slurry to bubble the slurry in the raw liquid vat. WO 2004/076394 discloses a process for recovering a solid adduct of a bis( 4-hydroxyaryl)alkane and a phenolic compound from a suspension comprising the adduct, wherein the process comprises the steps of a) supplying the suspension to a rotary filter, b) filtering the supplied suspension in the rotary filter to retain adduct as an adduct cake, c) pre-drying the adduct cake with an inert gas, d) washing the pre-dried adduct cake, e) optionally drying the washed adduct cake, and f) discharging the washed adduct cake from the rotary filter.

An improved rotary vacuum filter unit for the production of bisphenol A would be desirable.

BRIEF DESCRIPTION

In accordance with the invention there is provided a rotary vacuum filter as defined in claim 1 with preferred embodimente being defined in the dependent claims. Further provided is a filter unit as defined in claim 13 and its dependent claim.

A filter unit can include the above-described rotary vacuum filter and a scraper comprising a scraper section proximate to said filter cloth for removing said bisphenol A-phenol adduct crystals from said filter cloth.

A method for separating bisphenol A-phenol adduct crystals from a crystallization liquor can include feeding a crystallized stream comprising the bisphenol A-phenol adduct crystals and the crystallization liquor to the above-described rotary vacuum filter or the above-described filter unit and separating the bisphenol A-phenol adduct crystals from the crystallization liquor to form a filter cake on said filter cloth and a crystallization liquor stream in said interior surface of said perforated sector.

The above described and other features are exemplified by the following figures, detailed description, examples, and claims.

BRIEF DESCRIPTION OF THE DRAWINGS

The following figures are exemplary embodiments wherein the like elements are numbered alike.

FIG. 1 is an illustration of an embodiment of a bisphenol A production facility.
FIG. 2 is an illustration of an embodiment of a filter unit.

FIG. 3 is an illustration of an embodiment of a filter drum assembly.

FIG. 4 is an illustration of cross-section A-A of the filter drum assembly illustrated in FIG. 3 illustrating a lateral groove with an insert and a connector.

FIG. 5 is an illustration of a cross-section of another filter drum assembly illustrating a lateral groove with an insert.

FIG. 6 is an illustration of a cross-section of yet another filter drum assembly illustrating a lateral groove with a spring.

FIG. 7 is an illustration of a cross-section of yet another filter drum assembly illustrating a lateral groove with a metallic piece and a screw.

FIG. 8 is an illustration of a top view of metallic piece 470, screws 480, and filter cloths 326, 328 from the filter drum assembly illustrated in FIG. 7.

FIG. 9A is a graphical illustration of the filtrate weight change as a function of time for Examples 6 and 9, and Comparative Examples 1A, 1B, and 2.

FIG. 9B is a graphical illustration of the filtrate weight change as a function of time for Examples 1, 3-5, and 10.

FIG. 10A is a graphical illustration of the change of the filtrate weight over the change in time as a function of time for Examples 6 and 9, and Comparative Examples 1A, 1B, and 2.

FIG. 10B is a graphical illustration of the change of the filtrate weight over the change in time as a function of time for Examples 1, 3-5 and 10.

FIG. 11A is a picture of the tensile strength test setup used in Examples 14-21.

FIG. 11B is an illustration of the tensile strength test setup used in Examples 14-21.

FIG. 12 is an illustration of the parameters used in the fitting pressure calculations for Examples 14-21.

The above described and other features are exemplified by the detailed description, claims, and examples.

DETAILED DESCRIPTION

Bisphenol A (BPA) production plants can comprise a filter unit including a rotary vacuum filter that can facilitate separation of the formed BPA-phenol adduct crystals from the crystallization liquor (i.e., mother liquor). It is noted that "adduct" as used herein refers to the physical association of bisphenol A and phenol (e.g., one mole of bisphenol A and one mole of phenol can crystallize together to form a 1:1 molar ratio of bisphenol A /phenol adduct). The BPA-phenol adduct crystals can be needle-shaped. A dimension (e.g., diameter, width, or length) of the BPA-phenol adduct crystals can be 80 micrometers to 200 micrometers. The rotary vacuum filter can include a filter cloth attached to a filter drum. The filter drum rotates over an agitated pan that maintains the BPA-phenol adduct crystals suspended in solution. Vacuum from inside of the filter drum facilitates formation of a filter cake of the BPA-phenol adduct crystal to form on the filter cloth by pulling crystallization liquor into the filter drum. A scraper is held proximate to (e.g., millimeters (mm) away from) the filter cloth for filter cake detachment and recovery.

The rotary vacuum filter can be subject to frequent shutdowns in order to replace worn, damaged, or detached polymeric filter cloths. For instance, the polymeric filter cloth can be damaged by the scraper, by the parts for attaching the polymeric filter cloth to the filter drum, or by abrasion between polymeric filter cloth layers. In addition, the polymeric filter cloth can be loosen from fitting grooves into which they are introduced for attachment to the filter drum. These issues can result in shutdown of the rotary vacuum filter, replacement of the polymeric filter cloth, and knife realignment, which can take amounts of time (e.g., approximately 6 days from shutting down to start up) and reduce productivity.

It was surprisingly found that modification of one or more of the following parameters: (1) filter cloth material, (2) filter cloth layout, (3) fastening (i.e., attaching) method, and (4) scraper material as described in the present disclosure, renders an improved rotary vacuum filter for separation of BPA-phenol adduct crystals from a crystallization liquor. For instance, an increase in the mean time between failures of the rotary vacuum filter and a decrease in the time for repairs can be achieved. An increase in the mean time between failures can also result in improvement of other production based metrics such as lower mass of product lost, lower phenol usages due to lower BPA in the feed to reactors, lower nitrogen usages, lower steam usages due to less washing of the rotary vacuum filter, and lower amounts of phenol in the dehydration area.

As used herein the term "mean time between failures" refers to the period of time between starting the rotary vacuum filtration process after a shutdown to the next shutdown of the rotary vacuum filter. As used herein the term "failure" refers to at least one of an amount of bisphenol A-phenol adduct crystals present in the crystallization liquor of equal to or greater than 25 weight %, based on the total weight of the crystallization liquor; and a fluctuation in the vacuum pressure with the same frequency as the filter drum rotations per minute (e.g., fluctuations in vacuum pressure occurring the same number of times per minute as the filter drum rotations per minute, which can indicate damage, e.g., holes, in the filter cloth). The failures can be due to wear or damage to the filter cloths, wear or damage to the scraper, or loosening of the filter cloths from the filter drum.

A rotary vacuum filter for separation of bisphenol A-phenol adduct crystals from a crystallization liquor was developed that surprisingly increases the mean time between failures. The rotary vacuum filter includes a filter drum with a perforated sector on a lateral surface of the filter drum, a non-polymeric filter cloth attached to the filter drum and covering the perforated sector (i.e., a filter drum assembly), and a vacuum pumps in fluid communication with the perforated sectors.

A rotary vacuum filter can include more than 1, or more than 2 filter drum assemblies. A filter drum can be made up of one or more perforated sectors on a lateral side of the filter drum. Desirably, a filter drum can include 6 or more perforated sectors, 10 or more perforated sectors, or 20 or more perforated sectors. The lateral side of the filter drum can have one or more annular grooves and one or more lateral grooves adjacent to the perforated sections.

The grooves can have an inside width that is greater than a surface width. For example, the groove can have a width at adjacent the surface of the drum (Wi), and a width below the surface of the drum (in the groove) ($W_2$), wherein $W_2 > Wi$; preferably, $W_2 \geq 1.1W_1$; or $W_2 \geq 1.2W_1$. With an inside width that is greater than the surface width, when pressure is applied to the insert (e.g., a thermoplastic elastomeric insert), the insert deforms, spreading within the groove, and more firmly anchoring the ends of the filter cloth in the groove.

Perforated sections can be covered with one or more filter cloths attached to the filter drum. Desirably, a filter drum assembly includes 2 or more filter cloths 224, 6 or more filter cloths, or 10 more filter cloths, or 20 or more filter cloths. For instance, each perforated sector can be covered by one filter cloth.

As used herein, "non-polymeric filter cloth" or "filter cloth" refers to any material through which a fluid can be filtered and comprising a material other than a thermoplastic polymer. The non-polymeric filter cloth can be metallic. For instance, the non-polymeric filter cloth can be stainless steel.

An attaching piece for attaching the filter cloth to the filter drum of a rotary vacuum filter was developed that surprisingly increases the mean time between failures. The attaching piece can be an insert in a groove adjacent to the perforated sectors, wherein at least a portion of the filter cloth is fixed in the groove by the insert abutting the portion of the filter cloth opposite an interior surface of the groove. The insert can be a thermoplastic elastomer or a metallic material. The insert can be a cord, such as a thermoplastic elastomer cord, a spring, a metallic cable, or a metallic piece. For instance, several metallic bars can be inserted end-to-end into a groove adjacent to the perforated sectors. The length of the metallic pieces can be in the range of 60 to 200 millimeters.

More than 1, or more than 2 inserts can be present in an annular groove or a lateral groove.

An attaching piece can be a connector that further assists in maintaining the insert in the groove, for example that increases the compression (e.g., fitting pressure) of the filter cloth against the interior surface of the grooves. The connector can be a bolting piece (e.g., a bolt, screw, rivot, bar, and so forth). One connector (e.g., bolting piece), more than 1, or more than 2, or more than 5, or more than 10 can be present in a groove or an attaching piece. For example, the insert can be compressed into the groove with multiple fasteners (e.g., connectors) that compress the insert.

The attaching pieces ensure that there is little or no relative movement between the filter drum and the filter cloth and allow for ease of removal to reduce the time for replacement of filter cloths.

The non-polymeric filter cloth can be woven or nonwoven. For instance, the non-polymeric filter cloth can be woven in a Dutch weave, a reverse Dutch weave, a heddle atlas weave, or a square weave. The non-polymeric filter cloth can be sintered fibers.

A pore size of the non-polymeric filter cloth can be 50 micrometers to 350 micrometers, or 150 micrometers to 250 micrometers.

A thickness of the non-polymeric filter cloth can be equal to or less than 1 millimeter (mm), e.g., 0.1 mm to 1 mm, preferably 0.25 mm to 0.75 mm. This thickness allows for bending the filter cloth and introducing the cloth into a groove of the filter drum for fastening of the non-polymeric filter cloth.

An average weight change of the crystallization liquor during separation of the bisphenol A-phenol adduct crystals from the crystallization liquor by the filter cloth can be equal to or greater than 4.0 grams per second (g/s). This permeability of the non-polymeric filter cloth can provide a low cake wetness and a low level of impurities in the separated material.

The filter cloth can have a tensile strength and elastic limit to allow for the bending and fastening of the non-polymeric filter cloth to a groove in the filter drum. For instance, the non-polymeric filter cloth can have a tensile strength equal to or greater than 400 Newtons per centimeter (N/cm), or equal to or greater than 500 N/cm, or equal to or greater than 600 N/cm in the machine direction, the cross direction, or both. The tensile strength can be measured by the ISO 527 test method.

The non-polymeric filter cloth can each cover one sector or cover multiple sectors. Thus, if wear or damage (such as a hole) is detected in one sector, that piece of non-polymeric filter cloth can be removed while keeping the other filter cloths in place.

The non-polymeric filter cloth can be attached to the filter drum in a single layer to avoid abrasion between multiple layers.

One vacuum pump, more than one, or more than 2 vacuum pumps can be present in a rotary vacuum filter.

The mean time between failures of the non-polymeric filter cloth for separation of bisphenol A-phenol adduct crystals from a crystallization liquor can be greater than or equal to 500 days, or greater than or equal to 2 years, or greater than or equal to 3 years, or greater than or equal to 4 years, or greater than or equal to 5 years. The mean time between failures of the non-polymeric filter cloth for separation of bisphenol A-phenol adduct crystals from a crystallization liquor can be 500 days to 5 years, or 2 to 5 years.4

The mean time between failures of the rotary vacuum filter for separation of bisphenol A-phenol adduct crystals from a crystallization liquor can be greater than or equal to 500 days, or greater than or equal to 2 years, or greater than or equal to 3 years, or greater than or equal to 4 years, or greater than or equal to 5 years. The mean time between failures of the rotary vacuum filter for separation of bisphenol A-phenol adduct crystals from a crystallization liquor can be 500 days to 5 years, or 2 to 5 years.

A filter unit for separation of bisphenol A-phenol adduct crystals from a crystallization liquor was developed that surprisingly increases the mean time between failures. The filter unit includes the above-described rotary vacuum filter and a scraper comprising a scraper section proximate to the filter cloth for removing said bisphenol A-phenol adduct crystals from the filter cloth.

The scraper section can include a polymer selected from at least one of polyether ether ketone or polytetrafluoroethylene, preferably comprises both polyether ether ketone and polytetrafluoroethylene. The scraper section can include a polyether ether ketone. The scraper section can include polytetrafluoroethylene. The polymer can include a filler, such as at least one of glass fiber, including textile glass fibers such as E, A, C, ECR, R, S, D, NE glasses, or quartz. The glass fiber can be present in the polymer in an amount equal to or less than 30 wt.%.

A Mohs hardness of the scraper can be less than a Mohs hardness of the filter cloth. As the scraper is softer than the non-polymeric filter cloth, the scraper can wear faster than the non-polymeric filter cloth. Changing the scraper can take less time than changing the filter cloth, and thus, time for repairs can be reduced. In addition, a softer scraper can reduce damage to the filter cloth by the scraper itself.

One scraper, more than one scraper, or more than 2 scrapers or scraper sections can be present. Desirably, 1 to 5 scrapers can be present. When more than one scraper is used, the scrapers can be positioned back-to-back (e.g., to provide a double edge) or at various positions or angles proximate to the filter drum assembly.

The use of more than one scraper section, preferably 2 to 5 scraper sections, allows for ease of alignment of the scraper relative to the filter drum assembly, as each scraper section can be separately adjusted and aligned proximate to the filter drum assembly in order to remove the filter cake from the non-polymeric filter cloth. One or more scraper sections can be replaced when damaged to avoid replacing the whole scraper. Thus, the undamaged portions of the scraper can be further utilized and less material discarded or wasted. The scraper sections can be attached along the scraper length (and along the lateral surface of the filter drum) end-to-end adjacent to one another by bolting each section to a bar proximate to and traversing the lateral surface of the filter drum.

As used herein, "scraper" refers to any article that can be used to physically remove the filter cake from the filter cloth, such as a blade or a knife.

A method for separating bisphenol A-phenol adduct crystals from a crystallization liquor can include feeding a crystallized stream into the above-described rotary vacuum filter or the above-described filter unit, and separating the bisphenol A-phenol adduct crystals from the crystallization liquor to form a filter cake on said filter cloth and a crystallization liquor stream. With the presently disclosed filter a mean time between failures of the rotary vacuum filter is increased as compared to a unitary cloth. With the present filter, the mean time between failures can be equal to or greater than 500 days, e.g., greater than or equal to 18 months, greater than or equal to 24 months, or greater than or equal to 36 months.

The filter unit can comprise two or more filter units operating in different lines. For example, a crystallized stream can be split into two or more portions and each respective portion directed to a separate filter unit.

A portion of the crystallization liquor produced by the rotary vacuum filter can be combined with phenol and acetone before the mixed stream is fed into a bisphenol A reactor. An example of such an alternate process for recycling filtrate is described in U.S. Patent Application Publication 2013/0221837 A1, which is incorporated in its entirety herein by reference.

The product mixture used to form the crystallized stream can comprise one or more of 15 to 40 wt%, or 20 to 35 wt% of bisphenol A; 60 to 85 wt%, or 55 to 70 wt% of phenol; 5 to 15 wt% of byproduct; 0 to 5 wt%, or 0 to 3.5 wt% of water; and 0 to 8 wt%, or 0 to 1.5 wt% of acetone; all based on a total weight of the product mixture.

Use of the above-described rotary vacuum filter or the above-described filter unit for separating bisphenol A-phenol adduct crystals from a crystallization liquor is provided.

A more complete understanding of the components, processes, and apparatuses disclosed herein can be obtained by reference to the accompanying drawings. These figures (also referred to herein as "FIG.") are merely schematic representations based on convenience and the ease of demonstrating the present disclosure, and are, therefore, not intended to indicate relative size and dimensions of the devices or components thereof and/or to define or limit the scope of the exemplary embodiments. Although specific terms are used in the following description for the sake of clarity, these terms are intended to refer only to the particular structure of the embodiments selected for illustration in the drawings, and are not intended to define or limit the scope of the disclosure. In the drawings and the following description below, it is to be understood that like numeric designations refer to components of like function.

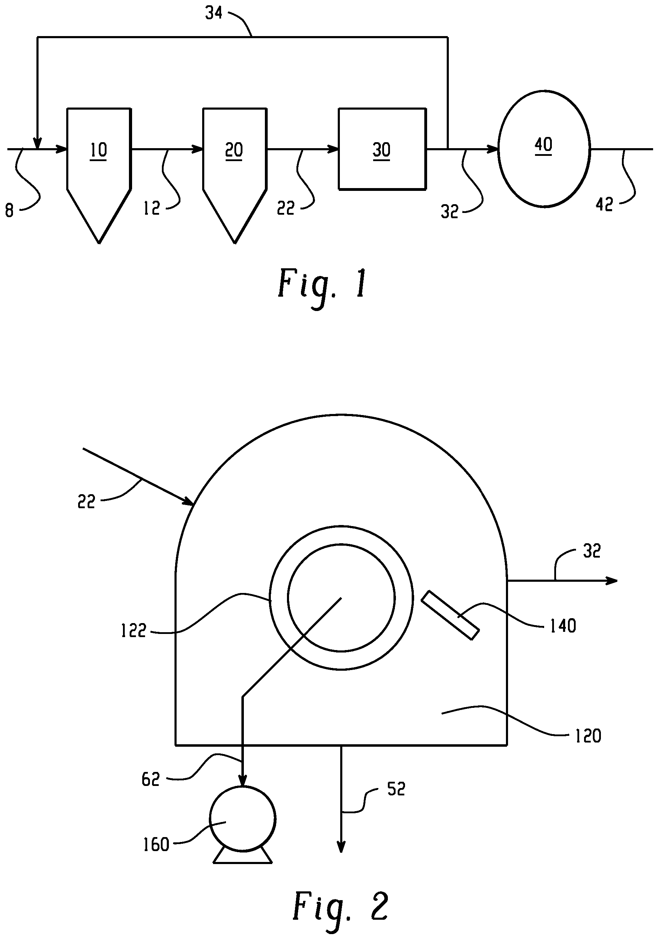

As illustrated in FIG. 1, a bisphenol A production facility can include reactor feed stream 8 directed to bisphenol A reactor 10 to form bisphenol A stream 12. Reactor feed stream 8 can comprise phenol, acetone, and optionally a promoter. Bisphenol A reactor 10 can be a fixed bed reactor comprising a catalyst. The phenol and acetone can be present in an amount of 5 to 15 moles of phenol per mole of acetone. Reactor feed stream 8 can comprise 75 to 95 weight percent (wt%) phenol and 1 to 8 wt% acetone. The phenol and acetone can be combined in a formulation tank located upstream of bisphenol A reactor 10. A portion of the crystallization liquor 34 from a downstream filtration process, such as at filter unit 30, can be combined with the phenol and acetone before the mixed stream is fed into bisphenol A reactor 10. Bisphenol A stream 12 can be removed from bisphenol A reactor 10.

Bisphenol A stream 12 comprises the product mixture from the bisphenol A reaction.

Bisphenol A stream 12 comprising the product mixture can be directed to crystallization unit 20 to form bisphenol A crystals comprising, for example, one or both of crystalline bisphenol A and an adduct of bisphenol A and phenol. The crystallization unit can comprise two or more crystallization units 20 operating in different lines. For example, the bisphenol A stream can be split into two or more portions and directing each respective portion to a separate crystallization unit. The crystals can be separated by removing the solid portion from the crystallization unit 20 comprising the crystals, for example, via filtration. To do so, crystallized stream 22 can be directed to filter unit 30 to form a crystallization liquor and a filter cake.

As illustrated in FIG. 2, crystallized stream 22 can be fed to rotary vacuum filter 120 of a filter unit. Rotary vacuum filter 120 can include filter drum assembly 122 in fluid communication with vacuum pump 160, which provides vacuum 62 to filter drum assembly 122. Proximate filter drum assembly 122 is scraper 140. As filter cake stream 32 is formed on the filter drum assembly, crystallization liquor 52 is formed by the vacuum in filter drum assembly 122.

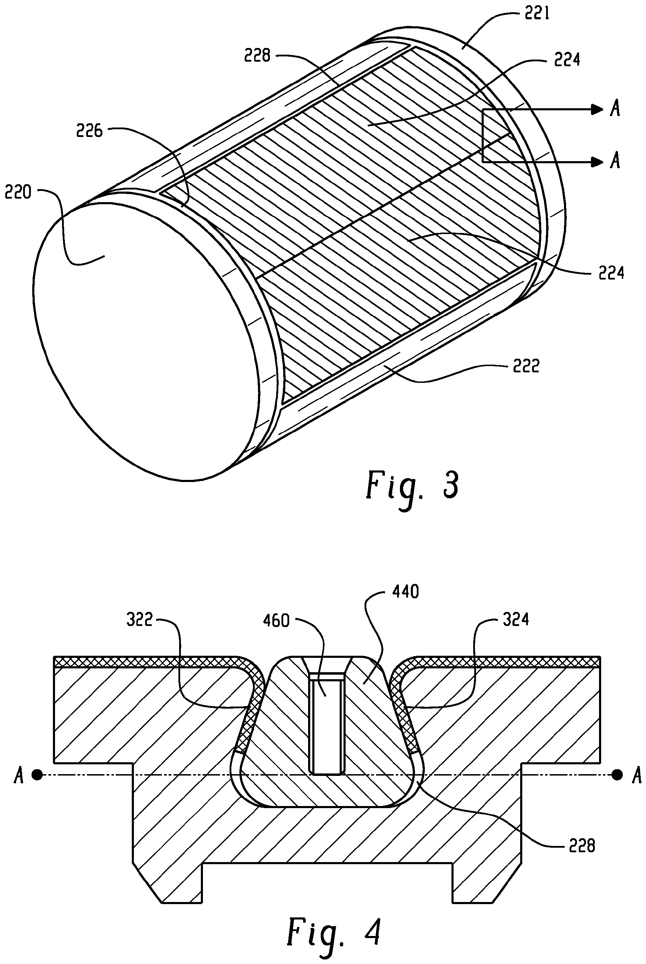

As illustrated in FIG. 3, filter drum 220 can be made up of a plurality of perforated sectors 222 on lateral side 221 of filter drum 220. Lateral side 221 can have annular grooves 226 and lateral grooves 228 adjacent to perforated sections 222. Perforated sections 222 can be covered with filter cloths 224 attached to filter drum 220.

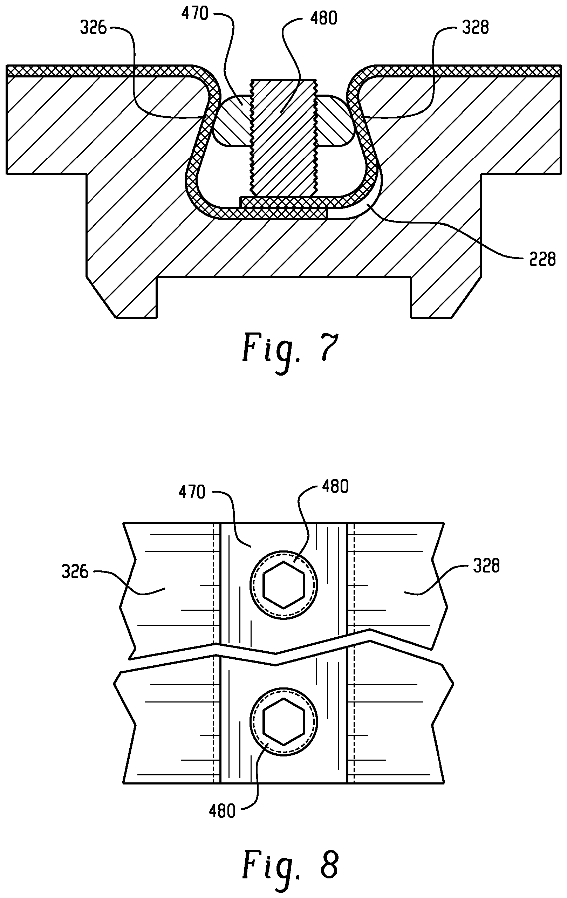

As illustrated in cross-section A-A of FIG. 4, a portion of first filter cloth 322 can be bent and introduced into lateral groove 228 on one side. A portion of second filter cloth 324 can be bent and introduced into lateral groove 228 on the opposite side. Insert 440 can be introduced into groove 228, abutting and compressing against portion of first filter cloth 322 and portion of second filter cloth 324 towards the sides of lateral groove 228. Connector (e.g., bolting piece) 460 can be introduced into a portion of insert 440 to apply further compression by insert 440 on portion of first filter cloth 322 and portion of second filter cloth 324. Bolting piece 460 can be a bolt, screw, rivet, bar, etc., (preferably a bolt or screw) introduced to expand insert 440 to increase the compression towards the sides of lateral groove 228. Preferably an end of the first filter cloth overlaps an end of the second filter cloth (see FIG. 7), e.g., such that the insert 440 further presses the ends of the filter cloths together, thereby further securing them in the groove.

Thus, in FIG. 3 filter cloths 224 are bent around their perimeters into the corresponding annular grooves 226 and lateral grooves 228 for attachment to filter drum 220 using inserts, bolting pieces, or both.

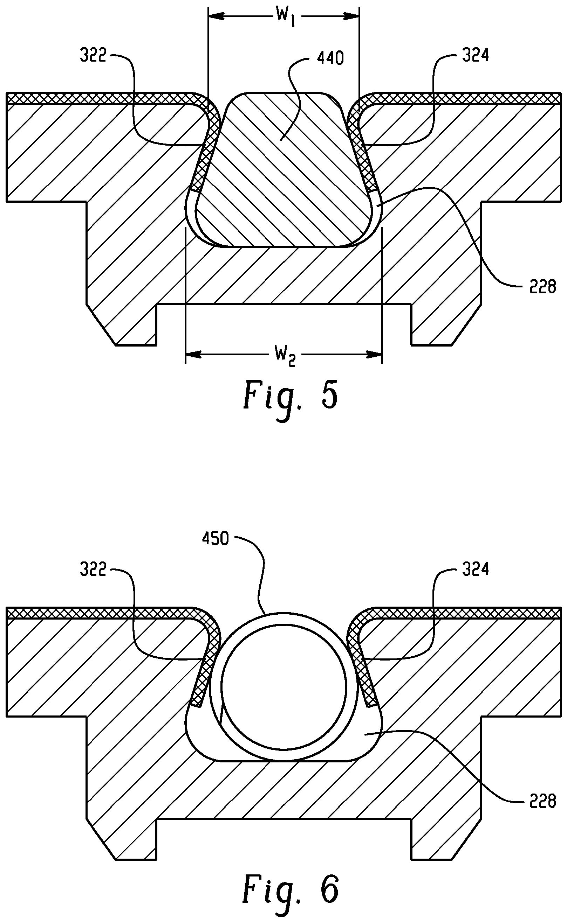

As illustrated in FIG. 5, an alternate filter drum assembly does not include bolting piece 460.

As illustrated in FIG. 6, another filter drum assembly includes spring 450 introduced into groove 228 instead of insert 440.

As illustrated in FIG. 7, still another filter drum assembly includes a metallic piece 470 in the shape of a bar instead of insert 440. As illustrated in FIG. 8, connector (e.g., a bolt, screw, rivet, bar, or the like), e.g., screws 480 can be inserted into metallic piece 470 along its length. When the screws 480 are screwed into metallic piece 470, the cross-sectional profile of metallic piece 470 increases the compress against portion of first cloth 326 and portion of second cloth 328, achieving a higher compression than the compression exerted by at least some other attaching pieces.

As illustrated in FIG. 1, filter cake stream 32 can be directed to melting unit 40 to form melted stream 42. The melting unit 40 can melt the crystals, for example, by heating the crystals at a temperature greater than the crystallization temperature. An additional amount of phenol can be added to the filter cake stream 32 to facilitate the melting of the crystals at a lower temperature. The melted stream can be further purified to produce a product bisphenol A. The product bisphenol A can be solidified, for example, in a flaking unit or a prilling tower (not shown in FIG. 1).

This disclosure is further illustrated by the following examples, which are nonlimiting.

EXAMPLES

Exemplary stainless steel filter cloths and their characteristics are summarized below in Table 1.

| Table 1 | | | | | | | | | | |
|---|---|---|---|---|---|---|---|---|---|---|
| Sample | 1 | 2 | 3 | 4 | 5 | 6 | 7 | 8 | 9 | 10 |
| Type | 30 warp x 150 weft | 24 warp x 110 weft | 150S | 200S | TELA™ 5110/140 | 12 warp x 64 weft | 98 warp x 98 weft | 78 warp x 78 weft | 49 warp x 49 weft | |
| Weave | Plain Dutch Weave | Plain Dutch Weave | Reverse Plain Dutch Weave | Reverse Plain Dutch Weave | Heddle Atlas Weave | Plain Dutch Weave | Square Weave | Square Weave | Square Weave | Sintered Fiber |
| Geometrical Pore Size (micrometers) | 122 | 153 | 144 | 148 | 98 | 301 | 160 | 200 | 315 | 58 |
| Tensile Strength Warp (N/cm) | 423 | 756 | 1267 | 1260 | - | 750 | - | - | - | - |
| Tensile Strength Weft (N/cm) | 750 | 948 | 1006 | 914 | - | 2620 | - | - | - | - |
| Thickness (mm) | 0.503 | 0.75 | 0.922 | 0.932 | 0.426 | 1.21 | 0.221 | 0.262 | 0.411 | - |
| Cloth weight (kilogram per meter squared (kg/m$^2$)) | 1.53 | 2.59 | 3.11 | 3.09 | 1.28 | 4.1 | 0.49 | 0.59 | 0.96 | - |

Examples 1-7 and Comparative Examples A-B: Filtration cloth permeability

Seven filter cloths were tested for their filtration rate of *para-para-bisphenol* A-phenol (ppBPA-phenol) adduct crystals in phenol. In addition, two comparative filter cloths were tested. Comparative Example A was a polyether ether ketone (PEEK) filter cloth. Comparative Example B was two layers of polypropylene (PP) filter cloth. The properties of these filter clothes is summarized in Table 2.

| Table 2 | | | |
|---|---|---|---|
| | PEEK filter cloth | PP filter cloth | |
| Manufacturer | SEFAR | PURE AIR, INC. | |
| Material Style | (pre-shrunk) | 8.5 ounce, 12 mil (0.305 mm) | 15 mil (0.381 mm) |

The ppBPA-phenol adduct crystals were prepared by crystallizing 390 grams (g) of reactor effluent at 70°C (with 1 wt% seeding) and cooled to 56°C. The slurry formed provides a filter cake approximately 10 centimeters (cm) thick when filtered with a laboratory pocket filter having a heated jacket and drain point. An overpressure of 0.05 bar (5 kiloPascals (kPa)) at 56°C was used. The filtrate flow leaving the filter was measured every two seconds using a scale connected to an appropriate data acquisition software. Measurements on the filtered liquid filtrate (permeate) show that ppBPA content is approximately 12 to 13 weight percent for all the tested filtrates.

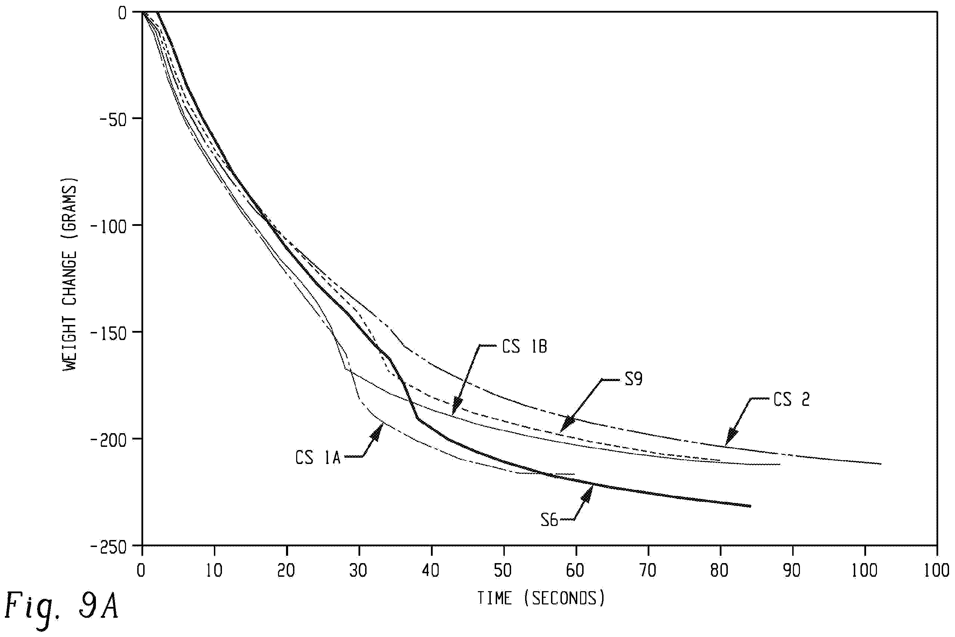

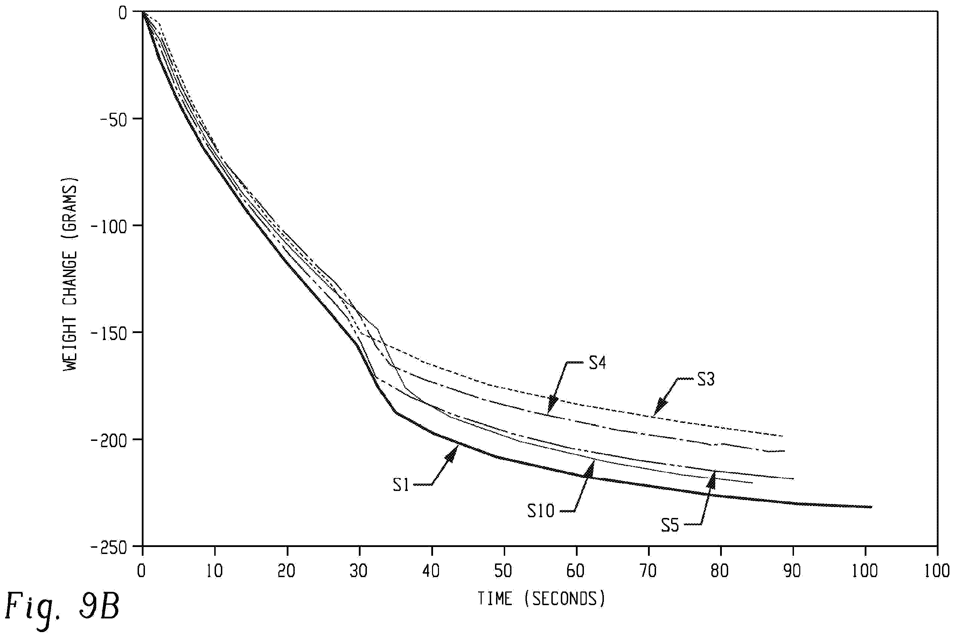

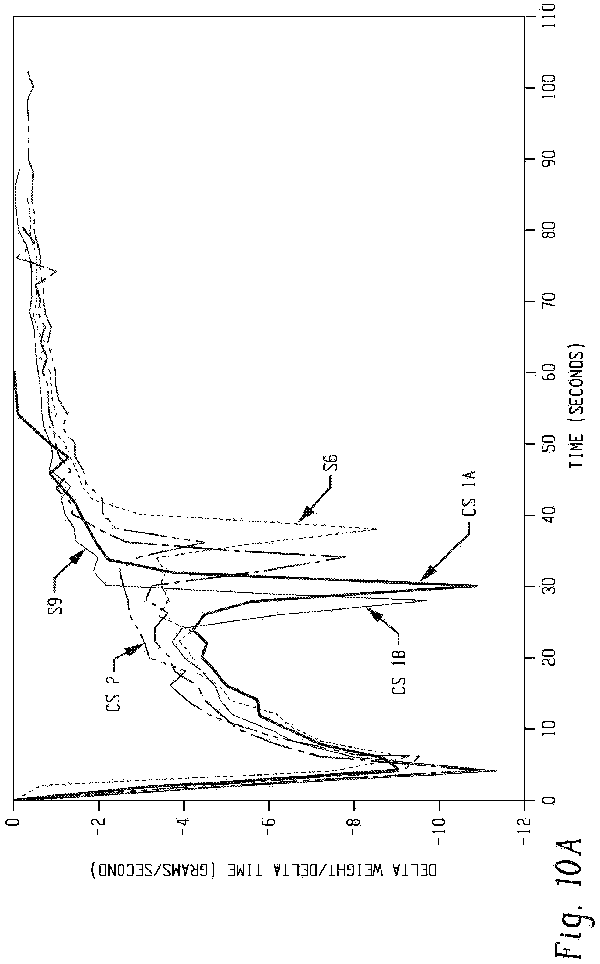

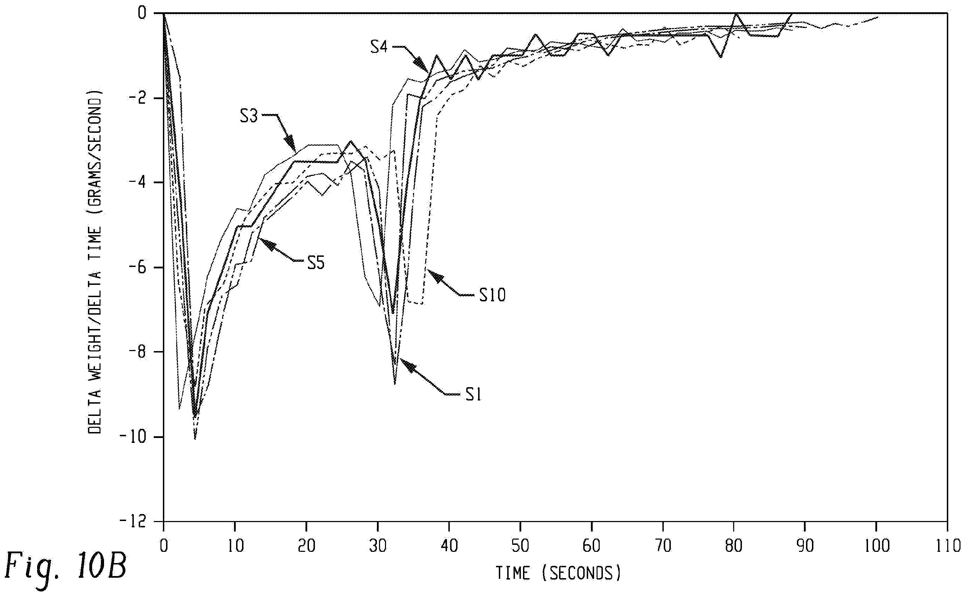

FIGs. 9 and 10 show a first phase (Phase 1) with a greater drop in filtrate weight over time followed by another phase (Phase 2) with a lower change in filtrate weight over time. Noticeably in FIG. 9 there is a dip in the data trend when Phase 1 ends and Phase 2 begins.

The time period of Phase 1, the average filtrate weight change and standard deviation in Phase 1 and Phase 2, and the concentration of ppBPA-phenol adduct crystals in the filtrate for the samples tested, is summarized in Table 3. As the cake generated is the same for each example, the filter cloth is the variable correlating to the different results. Thus, the average filtrate weight change provides a measure of the filtration performance.

| Table 3 | | | | | | |
|---|---|---|---|---|---|---|
| Sample | Phase 1 time (seconds)[1] | Phase 1 average weightdrop (g/s) | Phase 1 standard deviation of weight drop (g/s) | Phase 2 average weightdrop (g/s) | Phase 2 standard deviation of weight drop (g/s) | Concentration of ppBPA-phenol adduct crystals in filtrate (wt%) |
| Comparative Sample A. 1 | 30 | -5.55 | 1.35 | -1.33 | 0.95 | 12.9* |
| Comparative Sample A.2 | 28 | -5.21 | 1.30 | -0.83 | 0.55 | |
| Comparative Sample B | 36 | -3.63 | 1.25 | -0.85 | 0.57 | 13.0 |
| Sample 1 | 32 | -4.85 | 1.32 | -0.85 | 1.04 | 12.7 |
| Sample 5 | 32 | -5.05 | 1.56 | -0.82 | 0.48 | 13.0 |
| Sample 6 | 38 | -4.62 | 1.26 | -0.88 | 0.61 | 12.9 |
| Sample 9 | 34 | -4.42 | 1.27 | -0.91 | 0.55 | 13.0 |
| Sample 10 | 36 | -4.39 | 1.32 | -0.91 | 0.53 | 12.9 |
| Sample 3 | 30 | -4.50 | 1.46 | -0.82 | 0.44 | 13.3 |
| Sample 4 | 32 | -4.38 | 1.18 | -0.88 | 0.75 | 13.0 |
| * Average for repetitions A. 1 and A.2 of Comparative Sample A. [1] The time of Phase 1 until the start of Phase 2. | | | | | | |

The summarized data in Table 3 shows that the suitability of these non-polymeric filter cloths for filtering ppBPA-phenol adduct crystals, as they provide an acceptable permeability and do not allow for a loss in fines into the filtrate. Conversely if a longer time period for Phase 1 was observed, coupled with very small flows on Phase 2 and Phase 1, the tested cloths would have been unacceptable for phenol-BPA adduct crystals filtering. Not to be bound by theory, the differences obtained in the flows for both phases are very small, thus cloths behave with respect to filtering in the same way, i.e., the cake is the most important factor (rather than the cloth itself).

Examples 8-10: Chemical resistance of insert used to fix the filter cloth to the drum

Bisphenol A reactor effluent was contacted with samples of a SANTOPRENE™ 201-73 thermoplastic elastomer supplied by Eagle Burgmann Ibérica S.A, which can be used as the material for attaching piece inserts. The material was exposed to process fluids and temperature of 80°C. The insert material was measured and observed before and after exposure to determine if changes in length and weight occurred.

The insert was submerged in three different process stream solutions: (1) bisphenol A reactor effluent containing approximately 23 wt% of bisphenol A, 65 wt% of phenol, and 12 wt% of byproduct; all based on a total weight of the effluent;, (2) bisphenol A reactor effluent + 5 wt% acetone; and (3) phenol and 5 wt% acetone. Mechanical stresses such as those associated with use on a rotary vacuum filter was induced on the insert by introducing bolting pieces into them. The cloths on top of the inserts were mechanically fixed. The original and resultant dimensions of the insert material are shown in Table 4.

| Table 4 | | | | | | |
|---|---|---|---|---|---|---|
| | bisphenol A reactor effluent | | bisphenol A reactor effluent and 5 wt% acetone | | Phenol and 5 wt% acetone | |
| Time (hrs) | Weight (g) | Length (mm) | Weight (g) | Length (mm) | Weight (g) | Length (mm) |
| 0 | 31.57 | 22.8 | 26.66 | 19.2 | 21.81 | 15.6 |
| 168 | 32.53 | 22.8 | 27.2 | 19.2 | 21.85 | 15.6 |
| 360 | 32.51 | 22.8 | 27.81 | 19.2 | 21.81 | 15.6 |
| 504 | 33.30 | 22.8 | 27.70 | 19.2 | 21.86 | 15.6 |

The results show almost no change in length and weight of the insert material. The inserts did not change in appearance nor were other visual defects observable after 500 hours of testing.

Examples 11-21: Non-polymeric filter cloth fastening

In Examples 11-13, filter cloth Samples 1-3 were each fastened over three perforated sectors of a rotary vacuum filter drum using the attaching pieces illustrated in FIG. 4. The perforations were 0.5 centimeter circular perforations spaced 0.2 centimeters apart. Sample 1 could not be adjusted to conform to the curvature defects of the filter drum as well as Sample 2 and Sample 3. As such, Sample 1 was not in contact with the perforated sectors on some areas of the filter drum.

The filter cloths were covered with tape to simulate the effect of a filter cake on the filter cloths. As the tape has a lower porosity than a bisphenol A-phenol adduct filter cake, the measured cloth movement was greater than in operation. A pressure test was performed to check the cloth movement when the covered sector was pressurized, simulating the filter material behavior during a blowback phase.

A pressure regulator and manometer were used to maintain a constant pressure inside the sectors of 2 bars. The cloth displacement was measured by placing dial indicators on the filter cloth at three different sections: (1) close to the air intake; (2) at the center of the drum, and (3) in between sections (1) and section (2). At the beginning of every run the dial indicators were set to zero.

For all the filter cloths, movement was lowest at Section 1, which was closest to the annular strip and the air intake. For all the Samples, the location with the greatest amount of displacement (e.g., displacement of the filter cloth in the perpendicular direction with respect to the drum) was at the center of the drum length and in the center of the perforated sector, which is furthest away from the annular grooves and the lateral grooves. The filter cloth with the lowest movement was Sample 2 and filter cloth with the most movement was Sample 1.

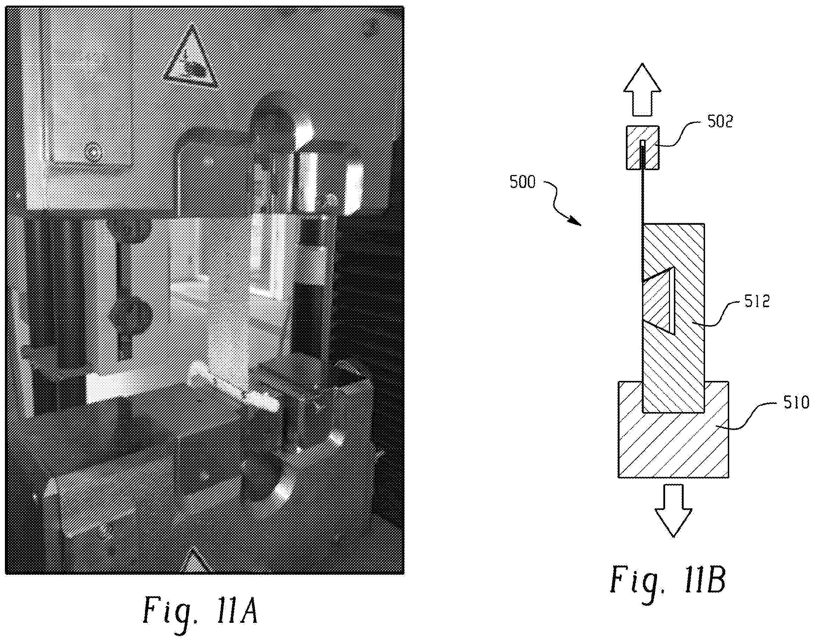

In Examples 14-21, the attaching pieces illustrated in FIG. 4 (Examples 14-20) and FIG. 7 (Example 21) were tested for fitting pressure (i.e., compression of the filter cloths by the attaching pieces) using the test setup shown in FIGs. 11A-B. As illustrated in FIG. 11B, each test assembly 500 includes test filter drum assembly 512 positioned on its end in holder 510. Holder 510 and filter cloth holder 502 exert a tensile force on test filter drum assembly 512.

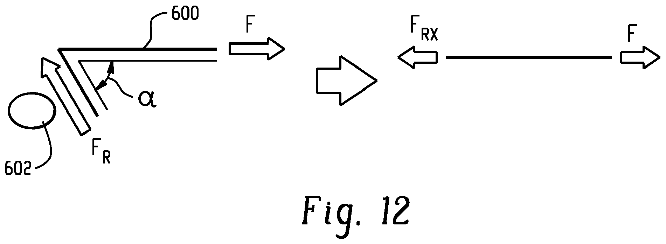

The resulting fitting pressure was calculated using Equation (1), Equation (2), and Equation (3). FIG. 12 illustrates the parameters used in Equations (1)-(3) in relation to filter cloth 600 and attaching piece 602. Tensile force upward (F) is equal to tensile force downward ($F_{Rx}$).

$$F_R = F_{Rx}/\sin(\alpha) \qquad \text{Equation (1)}$$

$$F_R = \mu \times N \qquad \text{Equation (2)}$$

$$\tau = N/A_d \qquad \text{Equation (3)}$$

where $F_R$ is the friction force between the attaching piece and the filter cloth, N is the normal force, $\mu$ is the coefficient of friction of the filter cloth, $\tau$ is the fitting pressure, and $A_d$ is the contact area between the attaching piece and the filter cloth.

The effect of temperature and phenol contact was tested in Examples 14-20. The fitting pressures exerted by the attaching pieces illustrated in FIG. 4 and FIG. 7 were compared in Examples 14 and 21. The results of Examples 14-21 are shown on Table 5. No significant differences were seen between Examples 14-20. As it can be seen in the results, the fitting pressure for the attaching pieces of FIG. 7 was much higher than the fitting pressure of the attaching pieces of FIG. 4. Therefore, the fatigue stress over the filter cloth can be higher without the risk of loosening the filter cloth from the drum.

Examples 22-29: Filter unit testing

Eight filter unit assemblies (including filter cloth, attaching pieces, and scraper) were tested for mean time between failures of a rotary vacuum filter for separation of bisphenol A-phenol adduct crystals from a crystallization liquor. Test of these examples was performed using a rotary vacuum filter to filter a bisphenol A reactor effluent as described in Examples 8-10 to produce approximately 50 tons/hour (13 kilograms per second (kg/s)) to 75 tons/hour (19 kg/s) of filtrate. These examples are summarized in Table 6.

In some examples the scraper was divided in different sections. For instance, the scraper in Example 22 was made up of 5 sections along its length (and along the lateral surface of the filter drum) and each section was attached end-to-end adjacent to one another by bolting each section to a bar proximate to and traversing the lateral surface of the filter drum. Having the several sections helped with aligning the scraper to the filter drum.

The following observations were made: In Examples 22, 23, 24, and 25 there was filter cloth wear and the formation of holes, resulting in replacement of whole filter cloth. Also, the stainless steel wire came loose and broke down in Example 23. In Example 26 attaching the single piece filter cloth (a single cloth with two ends) made holes in the filter cloth near the grooves. As there were holes in the filter cloth, adequate separation of bisphenol A-phenol adduct crystals from a crystallization liquor could not be achieved properly. In Example 27, there was scraper wear. Examples 28-29 did not exhibit any of the foregoing problems.

Table 5

| | Example 14 | Example 15 | Example 16 | Example 17 | Example 18 | Example 19 | Example 20 | Example 21 |
|---|---|---|---|---|---|---|---|---|
| Attaching Pieces | Cord insert with bolts | Cord insert with bolts | Cord insert with bolts | Cord insert with bolts | Cord insert with bolts | Cord insert with bolts | Cord insert with bolts | Metallic bar with bolts |
| Test conditions | Ambient temperature Test done after assembly | Tested after 24 hours at 100°C | Tested after 150 hours at 45°C | Tested after 300 hours at 45°C | Tested after 150 hours in contact with phenol at 75°C | Tested after 300 hours in contact with phenol at 75°C | Tested after 600 hours in contact with phenol at 75°C | Ambient temperature Test done after assembly |
| Fitting pressure (kg/cm²) | 3.6 | 3.2 | 3.3 | 3.3 | 2.8 | 3.0 | 3.2 | 53.2 |

Table 6

| Item | Example 22 | Example 23 | Example 24 | Example 24.1 | Example 25 | Example 25.1 | Example 26 |
|---|---|---|---|---|---|---|---|
| Filter cloth | Single piece polyether ether ketone | Single piece polyether ether ketone | Single piece polypropylene (two layers) | Single piece polypropylene (two layers) | Single piece PP (two layers) | Single piece PP (two layers) | Single piece stainless steel 316L |
| Attaching Pieces | Springs and cord insert | Springs and cord insert + stainless steel wire | Cord insert | Cord insert | Springs and cord insert | Springs and cord insert | Springs and cord insert |
| Scraper | Stainless steel in 5 sections | Stainless steel in 5 sections | Polytetrafluoroethylene + glass fiber in 1 section | Polytetrafluoroethylene + glass fiber in 5 sections | Stainless steel in 1 sections | Stainless steel in 5 sections | Polytetrafluoroethylene + glass fiber in 1 section |
| Mean time between Failures | 2 months | 3 months | 6 months | 6 months | 15 months | 12 months | Not operable |

| Table 6 (continued) | | | |
|---|---|---|---|
| Item | Example 27 | Example 28 | Example 29 |
| Filter cloth | One piece per perforated sector (20 pieces total), stainless steel 316L | One piece per perforated sector (20 pieces total), stainless steel 316L | One piece per perforated sector (20 pieces total), stainless steel 316L |
| Attaching Piece | Cord insert with bolts (FIG. 4) | Cord insert with bolts (FIG. 4) | Metallic bar with bolts (FIG. 7) |
| Scraper | Polytetrafluoroethylene + glass fiber in 5 sections | Polyether ether ketone + polytetrafluoroethylene in 5 sections | Polyether ether ketone + polytetrafluoroethylene in 5 sections |
| Mean time between Failures | 2 years (projected) | 4 years (projected) | 8 years (projected) |

Thus, the rotary vacuum filters and filter units described herein can be used to an increase in the mean time between failures of the rotary vacuum filter and a decrease in the time for repairs can be achieved.

The compositions, methods, and articles can alternatively comprise, consist of, or consist essentially of, any appropriate materials, steps, or components herein disclosed. The compositions, methods, and articles can additionally, or alternatively, be formulated so as to be devoid, or substantially free, of any materials (or species), steps, or components, that are otherwise not necessary to the achievement of the function or objectives of the compositions, methods, and articles.

All ranges disclosed herein are inclusive of the endpoints, and the endpoints are independently combinable with each other (e.g., ranges of "up to 25 wt.%, or, more specifically, 5 wt.% to 20 wt.%", is inclusive of the endpoints and all intermediate values of the ranges of "5 wt.% to 25 wt.%," etc.). "Combinations" is inclusive of blends, mixtures, alloys, reaction products, and the like. The terms "first," "second," and the like, do not denote any order, quantity, or importance, but rather are used to distinguish one element from another. The terms "a" and "an" and "the" do not denote a limitation of quantity, and are to be construed to cover both the singular and the plural, unless otherwise indicated herein or clearly contradicted by context. "Or" means "and/or" unless clearly stated otherwise. Reference throughout the specification to "some embodiments", "an embodiment", and so forth, means that a particular element described in connection with the embodiment is included in at least one embodiment described herein, and may or may not be present in other embodiments. In addition, it is to be understood that the described elements may be combined in any suitable manner in the various embodiments.

Unless specified to the contrary herein, all test standards are the most recent standard in effect as of the filing date of this application, or, if priority is claimed, the filing date of the earliest priority application in which the test standard appears.

Unless defined otherwise, technical and scientific terms used herein have the same meaning as is commonly understood by one of skill in the art to which this application belongs.

## Claims

1. A rotary vacuum filter (120) for separation of bisphenol A-phenol adduct crystals from a crystallization liquor, the rotary vacuum filter (120) comprising:

   a filter drum (220) with a perforated sector (222) on a lateral surface of the filter drum (220);
   a filter cloth (224) attached to the filter drum (220) and covering said perforated sector (222);
   a first attaching piece for attaching said filter cloth (224) to the filter drum (220), wherein said first attaching piece comprises an insert (440, 450) positioned in a groove (228) adjacent to said perforated sector (222),
   wherein at least a portion of said filter cloth (224) is fixed in said groove (228) by said insert (440,450) abutting said portion of said filter cloth (224) opposite an interior surface of said groove (228);
   a second attaching piece comprising a connector introduced into a portion of said insert (440,450); and

a vacuum pump (160) in fluid communication with an interior surface of the perforated sector (222), **characterised in that**,
said filter cloth (224) is a non-polymeric filter cloth and said groove (228) has an inside width (W2) that is greater than a surface width (W1).

2. The rotary vacuum filter (120) of claim 1, wherein said filter cloth (224) is metallic, preferably stainless steel.

3. The rotary vacuum filter (120) of any one or more of the preceding claims, comprising two or more non-polymeric filter cloths (224) and two or more perforated sectors (222), wherein said nonpolymeric filter cloths (224) are each covering to one of said perforated sectors (222).

4. The rotary vacuum filter (12) of Claim 3, comprising 6 or more non-polymeric filter Cloths (224); preferably 10 or more non-polymeric filter cloths (224); or 20 or more non-polymeric filter cloths (224); preferably wherein each perforated sector (222) is covered by one non-polymeric filter cloth (224).

5. The rotary vacuum filter (120) of any one or more of the preceding claims, wherein $W_2 \geq 1.1W_1$; or $W_2 \geq 1.2W_1$.

6. The rotary vacuum filter (120) of any one or more of the preceding claims, wherein, in the groove (228), an end of one non-polymeric filter cloth (224) overlaps with an end of another nonpolymeric filter cloth (224); preferably wherein the end of one non-polymeric filter cloth (224) overlaps with the end of another non-polymeric filter cloth (224) at a base of the groove (228)and wherein the overlap is pressed into the drum by a connector (e.g., bolting piece; such as a bolt, screw, rivet, or bar; preferably a screw), e.g., that passes through the insert (440, 450).

7. The rotary vacuum filter (120) of any one or more of the preceding claims, wherein said filter cloth (224) is woven, preferably woven in a Dutch weave, a reverse Dutch weave, a heddle atlas weave, or a square weave.

8. The rotary vacuum filter (120) of any one or more of the preceding claims, wherein a pore size of said filter cloth (224) is 50 micrometers to 350 micrometers, or 150 micrometers to 250 micrometers.

9. The rotary vacuum filter (120) of any one or more of the preceding claims, wherein a thickness of said filter cloth (224) is equal to or less than 1 millimeter.

10. The rotary vacuum filter (120) of any one or more of the preceding claims, wherein one layer of said filter cloth (224) is covering said perforated sector.

11. The rotary vacuum filter (120) of any one or more of the preceding claims, wherein a cloth weight of said filter cloth (224) is equal to or greater than 0.40 kilograms per meter squared.

12. The rotary vacuum filter (120) of any one or more of the preceding claims, wherein the insert (440, 450) is a thermoplastic elastomer.

13. A filter unit (30) comprising:
the rotary vacuum filter (120) of any one or more of the preceding claims; and a scraper (140) comprising a scraper section proximate said filter cloth (224) for removing said bisphenol A-phenol adduct crystals from said filter cloth (224).

14. The filter unit of Claim 13, wherein said scraper (140) comprises at least one of polyether ether ketone and polytetrafluoroethylene.

15. A method for separating bisphenol A-phenol adduct crystals from a crystallization liquor, the method comprising:
feeding a crystallized stream comprising the bisphenol A-phenol adduct crystals and the crystallization liquor to the rotary vacuum filter (120) of any one or more of Claims 1 to 12 or the filter unit (30) of any one or more of Claims 13 to 14; and separating the bisphenol A-phenol adduct crystals from the crystallization liquor to form a filter cake on said filter cloth (224) and a crystallization liquor stream in said interior surface of said perforated sector (222) .

16. Use of the rotary vacuum filter (120) of any one or more of Claims 1 to 12 or the filter unit (30) of any one or more of Claims 13 to 14 to separate bisphenol A-phenol adduct crystals from a crystallization liquor.

**Patentansprüche**

1. Vakuumdrehfilter (120) zur Trennung von Bisphenol A-Phenol-Adduktkristallen von einer Kristallisationslauge, wobei der Vakuumdrehfilter (120) Folgendes umfasst:

   eine Filtertrommel (220) mit einem perforierten Sektor (222) auf einer seitlichen Oberfläche der Filtertrommel (220),
   ein Filtertuch (224), das an der Filtertrommel (220) befestigt ist und den perforierten Sektor (222) bedeckt,
   ein erstes Befestigungsstück zum Befestigen des Filtertuchs (224) an der Filtertrommel (220), wobei das erste Befestigungsstück einen Einsatz (440, 450) umfasst, der in einer Nut (228) neben dem perforierten Sektor (222) positioniert ist,
   wobei mindestens ein Teil des Filtertuchs (224) in der Nut (228) durch den Einsatz (440, 450), der an dem Teil des Filtertuchs (224) gegenüber einer Innenfläche der Nut (228) anliegt, fixiert ist,
   ein zweites Befestigungsstück, das einen Verbinder umfasst, der in einen Teil des Einsatzes (440, 450) eingeführt ist, und
   eine Vakuumpumpe (160) in Fluidverbindung mit einer Innenfläche des perforierten Sektors (222), **dadurch gekennzeichnet, dass**
   das Filtertuch (224) ein nichtpolymeres Filtertuch ist und die Nut (228) eine Innenbreite (W2) aufweist, die größer ist als eine Oberflächenbreite (W1).

2. Vakuumdrehfilter (120) nach Anspruch 1,
   wobei das Filtertuch (224) metallisch, bevorzugt Edelstahl ist.

3. Vakuumdrehfilter (120) nach einem oder mehreren der vorhergehenden Ansprüche, umfassend zwei oder mehr nichtpolymere Filtertücher (224) und zwei oder mehr perforierte Sektoren (222), wobei die nichtpolymeren Filtertücher (224) jeweils einen der perforierten Sektoren (222) bedecken.

4. Vakuumdrehfilter (12) nach Anspruch 3, umfassend 6 oder mehr nichtpolymere Filtertücher (224), bevorzugt 10 oder mehr nichtpolymere Filtertücher (224) oder 20 oder mehr nichtpolymere Filtertücher (224), bevorzugt wobei jeder perforierte Sektor (222) durch ein nichtpolymeres Filtertuch (224) bedeckt ist.

5. Vakuumdrehfilter (120) nach einem oder mehreren der vorhergehenden Ansprüche, wobei $W_2 \geq 1,1\, W_1$ oder $W_2 \geq 1,2\, W_1$.

6. Vakuumdrehfilter (120) nach einem oder mehreren der vorhergehenden Ansprüche, wobei in der Nut (228) ein Ende eines nichtpolymeren Filtertuchs (224) ein Ende eines anderen nichtpolymeren Filtertuchs (224) überlappt, bevorzugt wobei das Ende eines nichtpolymeren Filtertuchs (224) das Ende eines anderen nichtpolymeren Filtertuchs (224) an einem Grund der Nut (228) überlappt und wobei die Überlappung durch einen Verbinder (z. B. Bolzenstück, wie z. B. einen Bolzen, eine Schraube, einen Niet oder eine Stange, bevorzugt eine Schraube), z. B. der durch den Einsatz (440, 450) verläuft, in die Trommel gedrückt wird.

7. Vakuumdrehfilter (120) nach einem oder mehreren der vorhergehenden Ansprüche, wobei das Filtertuch (224) gewebt ist, bevorzugt in einer Tressenbindung, einer Panzertressenbindung, einer Schaft-Atlasbindung oder einer Quadratmaschenbindung gewebt ist.

8. Vakuumdrehfilter (120) nach einem oder mehreren der vorhergehenden Ansprüche, wobei eine Porengröße des Filtertuchs (224) 50 Mikrometer bis 350 Mikrometer oder 150 Mikrometer bis 250 Mikrometer beträgt.

9. Vakuumdrehfilter (120) nach einem oder mehreren der vorhergehenden Ansprüche, wobei eine Dicke des Filtertuchs (224) gleich oder weniger als 1 Millimeter ist.

10. Vakuumdrehfilter (120) nach einem oder mehreren der vorhergehenden Ansprüche, wobei eine Schicht des Filtertuchs (224) den perforierten Sektor bedeckt.

11. Vakuumdrehfilter (120) nach einem oder mehreren der vorhergehenden Ansprüche, wobei ein Tuchgewicht des Filtertuchs (224) gleich oder größer als 0,40 Kilogramm pro Quadratmeter ist.

12. Vakuumdrehfilter (120) nach einem oder mehreren der vorhergehenden Ansprüche, wobei der Einsatz (440, 450) ein thermoplastisches Elastomer ist.

13. Filtereinheit (30), umfassend:
den Vakuumdrehfilter (120) nach einem oder mehreren der vorhergehenden Ansprüche und einen Abstreifer (140), der einen Abstreiferabschnitt nahe dem Filtertuch (224) zum Entfernen der Bisphenol A-Phenol-Adduktkristalle von dem Filtertuch (224) umfasst.

14. Filtereinheit nach Anspruch 13, wobei der Abstreifer (140) mindestens eines von Polyetheretherketon und Polytetrafluorethylen umfasst.

15. Verfahren zum Trennen von Bisphenol A-Phenol-Adduktkristallen von einer Kristallisationslauge, wobei das Verfahren Folgendes umfasst: Zuführen eines kristallisierten Stroms, der die Bisphenol A-Phenol-Adduktkristalle und die Kristallisationslauge umfasst, in den Vakuumdrehfilter (120) nach einem oder mehreren der Ansprüche 1 bis 12 oder die Filtereinheit (30) nach einem oder mehreren der Ansprüche 13 bis 14 und Trennen der Bisphenol A-Phenol-Adduktkristalle von der Kristallisationslauge, um einen Filterkuchen auf dem Filtertuch (224) und einen Kristallisationslaugenstrom in der Innenfläche des perforierten Sektors (222) zu bilden.

16. Verwendung des Vakuumdrehfilters (120) nach einem oder mehreren der Ansprüche 1 bis 12 oder der Filtereinheit (30) nach einem oder mehreren der Ansprüche 13 bis 14, um Bisphenol A-Phenol-Adduktkristalle von einer Kristallisationslauge zu trennen.

**Revendications**

1. Filtre à vide rotatif (120) pour la séparation de cristaux d'adduit de bisphénol A-phénol à partir d'une liqueur de cristallisation, le filtre à vide rotatif (120) comprenant :

un tambour filtrant (220) avec un secteur perforé (222) sur une surface latérale du tambour filtrant (220) ;
un tissu filtrant (224) fixé au tambour filtrant (220) et recouvrant ledit secteur perforé (222) ;
une première pièce de fixation pour fixer ledit tissu filtrant (224) au tambour filtrant (220), dans laquelle ladite première pièce de fixation comprend un insert (440, 450) positionné dans une rainure (228) adjacente audit secteur perforé (222),
dans lequel au moins une partie dudit tissu filtrant (224) est fixée dans ladite rainure (228) par ledit insert (440, 450) en butée contre ladite partie dudit tissu filtrant (224) opposée à une surface intérieure de ladite rainure (228) ;
une seconde pièce de fixation comprenant un connecteur introduit dans une partie dudit insert (440, 450) ; et
une pompe à vide (160) en communication fluidique avec une surface intérieure du secteur perforé (222), **caractérisée en ce que**
ledit tissu filtrant (224) est un tissu filtrant non polymère et ladite rainure (228) a une largeur intérieure (W2) qui est supérieure à une largeur de surface (W1).

2. Filtre à vide rotatif (120) selon la revendication 1, dans lequel ledit tissu filtrant (224) est métallique, de préférence en acier inoxydable.

3. Filtre à vide rotatif (120) selon l'une quelconque ou plusieurs des revendications précédentes, comprenant deux ou plusieurs tissus filtrants non polymères (224) et deux ou plusieurs secteurs perforés (222), dans lequel lesdits tissus filtrants non polymères (224) recouvrent chacun desdits secteurs perforés (222).

4. Filtre à vide rotatif (12) selon la revendication 3, comprenant au moins 6 tissus filtrants non polymères (224) ; de préférence au moins 10 tissus filtrants non polymères (224) ; ou au moins 20 tissus filtrants non polymères (224) ; de préférence dans lequel chaque secteur perforé (222) est recouvert d'un tissu filtrant non polymère (224).

5. Filtre à vide rotatif (120) selon l'une quelconque ou plusieurs des revendications précédentes, dans lequel $W_2 \geq 1,1W_1$ ; ou $W_2 \geq 1,2W_1$.

6. Filtre à vide rotatif (120) selon l'une quelconque ou plusieurs des revendications précédentes, dans lequel, dans la rainure (228), une extrémité d'un tissu filtrant non polymère (224) chevauche une extrémité d'un autre tissu filtrant non polymère (224) ; de préférence dans lequel l'extrémité d'un tissu filtrant non polymère (224) chevauche l'extrémité d'un autre tissu filtrant non polymère (224) à une base de la rainure (228) et dans lequel le chevauchement est enfoncé dans le tambour par un connecteur (par exemple, une pièce de boulonnage ; telle qu'un boulon, une vis, un rivet ou une barre ; de préférence une vis), par exemple, qui traverse l'insert (440, 450).

7. Filtre à vide rotatif (120) selon l'une quelconque ou plusieurs des revendications précédentes, dans lequel ledittissu filtrant (224) est tissé, de préférence tisséselon un tissage hollandais, un tissage hollandais inversé, un tissage satin à lices ou un tissage carré.

8. Filtre à vide rotatif (120) selon l'une quelconque ou plusieurs des revendications précédentes, dans lequel une taille de pores dudit tissu filtrant (224) est de 50 micromètres à 350 micromètres, ou de 150 micromètres à 250 micromètres.

9. Filtre à vide rotatif (120) selon l'une quelconque ou plusieurs des revendications précédentes, dans lequel une épaisseur dudit tissu filtrant (224) est égale ou inférieure à 1 millimètre.

10. Filtre à vide rotatif (120) selon l'une quelconque ou plusieurs des revendications précédentes, dans lequel une couche dudit tissu filtrant (224) recouvre ledit secteur perforé.

11. Filtre à vide rotatif (120) selon l'une quelconque ou plusieurs des revendications précédentes, dans lequel un poids de tissu dudit tissu filtrant (224) est égal ou supérieur à 0,40 kilogramme par mètre carré.

12. Filtre à vide rotatif (120) selon l'une quelconque ou plusieurs des revendications précédentes, dans lequel l'insert (440, 450) est un élastomère thermoplastique.

13. Unité de filtre (30) comprenant :
le filtre à vide rotatif (120) selon l'une quelconque ou plusieurs des revendications précédentes ; et un grattoir (140) comprenant une section de grattoir à proximité dudit tissu filtrant (224) pour éliminer lesdits cristaux d'adduit de bisphénol A-phénol dudit tissu filtrant (224).

14. Unité de filtre selon la revendication 13, dans laquelle ledit grattoir (140) comprend au moins un élément parmi le polyétheréthercétone et le polytétrafluoroéthylène.

15. Procédé pour séparer des cristaux d'adduit de bisphénol A-phénol d'une liqueur de cristallisation, le procédé comprenant : l'alimentationd'un courant cristallisé comprenant les cristaux d'adduit de bisphénol A-phénol et la liqueur de cristallisation au filtre à vide rotatif (120) selon l'une ou plusieurs des revendications 1 à 12 ou à l'unité de filtre (30) selon l'une ou plusieurs des revendications 13 à 14 ; et la séparation des cristaux d'adduit de bisphénol A-phénol à partir de la liqueur de cristallisation pour former un gâteau de filtre sur ledit tissu filtrant (224) et un courant de liqueur de cristallisation dans ladite surface intérieure dudit secteur perforé (222).

16. Utilisation du filtre à vide rotatif (120) selon l'une ou plusieurs des revendications 1 à 12 ou de l'unité de filtre (30) selon l'une ou plusieurs des revendications 13 à 14 pour séparer les cristaux d'adduit de bisphénol A-phénol à partir d'une liqueur de cristallisation.

Fig. 1

Fig. 2

*Fig. 3*

*Fig. 4*

Fig. 5

Fig. 6

326    470    480    328

228

**Fig. 7**

470    480

326    328

480

**Fig. 8**

EP 3 634 601 B1

*Fig. 9A*

Fig. 9B

EP 3 634 601 B1

Fig. 10A

Fig. 10B

*Fig. 11B*

502

512

510

500

*Fig. 11A*

Fig. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 19961521, Rainer
- WO 02055175 A, Daniel
- GB 899165 A
- JP 2007203184 A
- WO 2004076394 A
- US 20130221837 A1